(19) Europäisches Patentamt
European Patent Office
Office européen des brevets

(11) Publication number: 0 197 659
B1

(12) # EUROPEAN PATENT SPECIFICATION

(45) Date of publication of the patent specification: 31.05.89

(51) Int. Cl.⁴: C 07 C 103/48, C 07 C 102/00 // B01J31/02

(21) Application number: 86301677.0

(22) Date of filling: 10.03.86

(54) **Process for synthesizing N-acetylglycine using novel promoters.**

(30) Priority: 05.04.85 US 720249

(43) Date of publication of application: 15.10.86 Bulletin 86/42

(45) Publication of the grant of the patent: 31.05.89 Bulletin 89/22

(84) Designated Contracting States: BE DE FR GB NL

(56) References cited:
EP-A-0 170 830
DE-A-2 115 985
DE-A-3 242 374
FR-A-2 523 576

(73) Proprietor: TEXACO DEVELOPMENT CORPORATION, 2000 Westchester Avenue, White Plains New York 10650 (US)

(72) Inventor: Lin, Jiang- Jen, 2617 Oak Meadow Drive, Round Rock Texas 78664 (US)

(74) Representative: Burnside, Michael, Urquhart- Dykes & Lord 91 Wimpole Street, London W1M 8AH (GB)

EP 0 197 659 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convension)

LIBER, STOCKHOLM 1989

## Description

This invention relates to the catalytic amidocarbonylation of aldehydes in the presence of amides which leads to the production of N-acyl-L-amino acids.

More particularly this invention leads to an improved process for production of N-acetylglycine, a possible intermediate for phenylanine or L-DOPA synthesis or for glycine production, wherein paraformaldehyde is reacted with an amide and CO in the presence of a cobalt-containing compound, in complex at a defined ratio with a tertiarylphosphine or a nitrile promoter and a solvent, at a temperature of at least 25°C and a pressure of at least 3.5 MPa (500 psi).

Early attempts were made without success to synthesize α-amino acids or derivatives thereof by reacting a Schiff base or a nitrile with carbon monoxide and hydrogen. [Bull. Chem. Soc. Japan 33 (160) 78]

U.S. Patent No. 3 766 266 discloses a method of producing an N-acyl-α-amino acid which comprises holding an aldehyde, an amide of a carboxylic acid and carbon monoxide at a temperature of 10° to 300°C and a pressure of at least 500 atm. in the presence of a carbonylation catalyst until said N-acyl-α-amino acid is formed. Dicobalt octacarbonyl was used as the catalyst, whith catalyst can optionally be promoted by unspecified phosphorus and nitrogen-containing compounds.

In Chem. Comm. 1540 (1971), Wakamatsu, et al. first discloses a cobalt-catalyzed reaction which gives various N-acylamino-acids from an aldehyde, an amide and carbon monoxide. N-acetylglycine was prepared at low yield from paraformaldehyde, using a dicobalt octacarbonyl catalyst.

An article by Parnaud, et al., in Journal of Molecular Catalysis, 6 (1979) 341 - 350, discusses the synthesis potential and the catalytic mechanism for the reaction wherein N-acyl-α-amino acids are produced by reacting an aldehyde, CO and amide in the presence of dicobalt octacarbonyl.

In amidocarbonylation, the aldehyde can be generated in situ from allyl alcohol, alkyl halide, oxiranes, alcohols and olefins followed by reaction with an amide and carbon monoxide to produce an N-acyl-α-amino acid.

A related patent, U. S. Patent No. 3 996 288 discloses that when an alcohol or certain of its ester derivatives is held at 50°C to 200°C and 10 to 500 atm. in the presence of hydrogen, carbon monoxide, the amide of a carboxylic acid and a carbonylation catalyst, an aldehyde having one more carbon atom than the alcohol or ester is formed in good yield. If the amide has at least one active hydrogen atom on its amide nitrogen, it further reacts with the aldehyde and carbon monoxide to form an N-acylamino acid.

Hirai, et al. discuss a process for combining the transition metal catalyzed isomerization of allyl alcohol to aldehyde and cobalt catalyzed amidocarbonylation to provide a route from allylic alcohols to N-acyl-α-amino acids. See Tetrahedron Letters, Vol. 23, No. 24, pp. 2491 - 2494, 1982.

U.S. Patent No. 4 264 515 discloses a process for obtaining terminal N-acyl-α-amino acids by a reaction catalyzed by a cobalt carbonylation catalyst wherein the aldehyde is produced in situ from olefins and a $CO/H_2$ mixture. An unsaturated vegetable oil or $C_8$ - $C_{30}$ mono olefinic compound is reacted with an amide, carbon monoxide and hydrogen in the presence of a cobalt catalyst. The process is operated in one step and provides for increased selectivity.

Japanese Kokai Tokkyo Koho JP-5 869 853 discloses a process for reacting allyl alcohol, acetamide, CO and H in the presence of co and a Group VIII metal compound and a solvent to produce N-acyl amino acids.

German offen DE-3 242 374 teaches a process for producing acetyl amino acids by contacting oxiranes with amides in the presence of CO and $H_2$ and a cobalt-containing catalyst using a Group I - IV metal-containing compound as promoter.

Among these disclosures, dicobalt octacarbonyl was generally used as the active catalyst. In the case of the paraformaldehyde, acetamide and CO reaction, the product, N-acetylglycine, (the smallest molecule in amino acid family) acts as a complexing agent to the cobalt catalyst. As a result, the cobalt catalyst was deactivated and a high CO and $H_2$ pressure was required.

The modification of the cobalt catalyst by a ligand, such as phosphine, amine or nitrile has been reported. It is known that trialkylphosphine can be used to complex with cobalt for synthesis gas reactions, such as hydroformylation or methanol homologation. For example, in Netherland Patent 7 606 138, Shell claimed the combination of cobalt and tertiary phosphine for ethanol synthesis selectively from methanol and $CO/H_2$ mixtures. Celanese utilized a catalyst comprising a cobalt carbonyl in complex with an organic nitrogen ligand, including nitriles, for the reaction of methanol with carbon monoxide and hydrogen, in U.S. Patent 4 201 868 and 4 168 391.

In U.S. Patents 4 209 467, 4 092 188 and 3 996 164 amine ligands including pyridine, 2-hydroxypyridine and cycloaliphatic amines were employed with dicobalt octacarbonyl for hydroformylation or carbonylation of olefins. The function of ligands was to stabilise the catalyst and increase the product selectivity.

In U.S. Patent 3 931 332, the importance of the cobalt and diamine promoter ratio has been demonstrated Increasing the added amount of a diamine-stabiliser added markedly reduced the reaction rate of hydroformylation. The smaller amount of ligand to cobalt is preferred with respect to reaction rate.

In Erdol and Kohle Erdgas, Petrochemic 35(1), 36, 1982 and J. Mol. Cat. 12 (1981) 113 - 119, there was evidence of amino acids and their derivatives acting as the ligand for transition metals.

In Journal of Organometallic Chemistry, 279, (1985), 203 - 214, Ojima disclosed the homogeneous binary systems for the synthesis of N-acyl-α-amino acids via (1) the isomerization-amidocarbonylation of allyl alcohol (2) the isomerisation-amidocarbonylation of oxiranes and (3) the hydroformylation-amidocarbonylation of

trifluoropropene. In the case of the trifluoropropene reactions, the highly regioselective syntheses of N-acetyltrifluorovaline and N-acetyltrifluoronorvaline in high yields was achieved by using $Co_2(CO)_8$-$Rh_6(CO)_{16}$ and $Co_2(Co)_8$ respectively as catalysts.

The amidocarbonylation-reaction of an aldehyde, amide and carbon monoxide to form amidoacid, involves the iodide-free cobalt catalyzed carbonylation of an aldehyde-acetamide adduct, under unusually mild reaction temperatures (ca 120°C) compared with cobaltcatalyzed hydroformylation or carbonylation. Our examples in this invention have demonstrated the importance of various ligands. For comparison, succinonitrile and tri-n-butylphosphine ligands aided the cobalt recovery and the product selectivity; diamine and acetonitrile (large amount used as solvent) adversely affected the reaction. Although these effects are not well understood, we believe that the choice of ligands is dependent on the strength of the ligand to cobalt complex and on the amount of ligands used. In the process of the instant invention ligands have been relied upon which either complexed with cobalt stronger than the amido acid product did and or which were weak and had no deactivating ability.

According to the present invention there is provided a process for the production of N-acyl-L-amino acids of the formula

$$\begin{array}{l} R^4\text{-}\underset{\displaystyle |}{C}H\text{-}CO_2H \\ \quad\; NR^5.CO.R^6 \end{array}$$

in which $R^4$, $R^5$ and $R^6$, which may be the same or different, are each hydrogen or a linear, branched or cyclic organic group, by reaction between an aldehyde of the formula

$R^4$-CHO

and an amide of the formula

$R^5NH.CO.R^6$

at a temperature of at least 25°C and a pressure of at least 3.5 MPa with carbon monoxide and hydrogen, in the presence of a cobalt catalyst, characterised in that the cobalt catalyst is promoted in an inert solvent by a tertiary phosphine of the formula

$R_3P$

where R represents an alkyl, aryl or alkaryl radical, or by a nitrile of the formula CN-$R^7$-CN where $R^7$ represents an alkylene radical or an aromatic group.

A preferred form of the invention comprises contacting a mixture of an aldehyde such as paraformaldehyde and carbon monoxide, hydrogen and an amide with a catalyst comprising a cobalt-containing compound in complex with the nitrile promoter or a cobalt-containing compound promoted by the tertiaryphosphine ligand, dissolved in a solvent at a low or moderate pressure and a temperature of at least 25°C for a period of about 1 to 10 hours.

The use of the defined nitrile complexed with cobalt for amidocarbonylation is novel, as is the use of the defined tertiaryphosphine ligand for amido acid synthesis, the latter allowing the reaction to be carried out very efficiently at pressures of less than 7 MPa (1000 psi).

In the narrower and more preferred practice of this invention N-acylglycine is prepared from a mixture of paraformaldehyde, an amide and syngas (carbon monoxide and hydrogen) by an improved process which comprises contacting said mixture with a catalyst system comprising a cobalt-containing compound in complex with the nitrile promoter or the tertiarylphosphine ligand at the defined molar ratio and a substantially inert solvent at a temperature of at least 25°C and a pressure of at least 3.5 MPa (500 psi) until substantial formation of the desired N-acetylglycine has been achieved.

The synthesis using the nitrile or trialkylphosphine complex can best be represented by the following Equation 1:

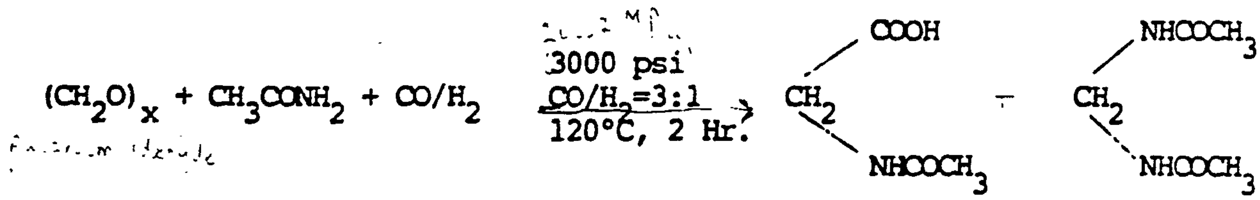


Recovery of N-acetylglycine from the reaction product can be carried out in any convenient or conventional manner such as by distillation, extraction, filtration, crystallization, etc.

The catalyst system suitable for the practice of the first embodiment of this invention comprises a cobalt-containing compound, complexed with nitrile in a substantially inert solvent.

In the catalyst system of this invention the cobalt-containing compound and succinonitrile are believed to be in a complex which allows the catalyst system to be highly active for amidocarbonylation and does not require high syngas pressure.

The cobalt-containing compound may take many different forms. For instance, the cobalt may be added to the reaction mixture in the form of a variety of inorganic or organic cobalt salts, or cobalt carbonyls. The cobalt may, for example, be added as a cobalt halide such as cobalt bromide or cobalt chloride, or it may be added as the salt of an aliphatic or aromatic carboxylic acid such as, for example, cobalt formate, cobalt acetate, cobalt butyrate, cobalt naphenate, and cobalt stearate. The cobalt carbonyl may be tetracobalt dodecacarbonyl or dicobalt octacarbonyl. The preferred cobalt-containing compound is dicobalt octacarbonyl.

Nitriles are used in one embodiment of the process of this invention to activate or stabilize the cobalt-containing compound. Comparative Examples II and III demonstrate that using no succinonitrile, the cobalt compound by itself demonstrated much lower selectivity to N-acetylglycine.

The nitrile is added in the form of structure A.

$$NC\text{-}R^7\text{-}CN$$
(A)

wherein $R^7$ represents an alkylene radical or an aromatic group. The preferred forms are adiponitrile, succinonitrile, glutaronitrile and ortho-phenylenediacetonitrile.

Tertiary phosphines are used in the second embodiment of the process of this invention to activate the cobalt-containing compound at pressures below 7 MPa (1000 psi). Comparative Example XI demonstrates that at pressures under 7 MPa (1000 psi), the cobalt compound by itself yielded no products.

Suitable tertiary phosphines which work in the process of the invention are represented by the formula:

$$R_3P$$

wherein R represents an alkyl, aryl or alkaryl radicals bonded to the phosphorous atom.

The organic radicals particularly useful include those having $C_1$ to $C_{10}$ carbon atoms in a linear chain; they include methyl, ethyl, butyl, heptyl, octyl and decyl. Methyl, butyl and ethyl are typical examples presently in commercial production. Especially useful is n-tributylphosphine.

Various aldehydes may be used as feedstock in the method of this invention. More specifically aliphatic, alicyclic, aromatic and heterocyclic aldehydes have been used successfully in the method of the invention. Aldehydes, giving good yields with suitable amides include paraformaldehyde, formaldehyde, trioxane, acetaldehyde, propionaldehyde, butyraldehyde, phenylacetaldehyde, 2,4-dihydroxyphenylacetaldehyde, indolylacetaldehyde, crotonaldehyde, β-formylpropionaldehyde, β-formylpropionic acid and its esters, β-methylmercaptopropionaldehyde, glycolaldehyde, β-acetoxypropionaldehyde, stearaldehyde, benzaldehyde, furfural, indolaldehyde, adipaldehyde and acrolein. Most preferred are paraformaldehyde and formaldehyde.

Suitable amide-containing coreactants that are useful in the amidocarbonylation reaction have the general structure:

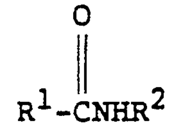

where the $R^1$, and $R^2$ groups may be aryl, alkyl, arylalkyl and alkylaryl radicals, or hydrogen, including the methyl, ethyl, butyl, n-octyl, phenyl, benzyl and chlorophenyl groupings. Examples of suitable amide coreactants include acetamide, benzamide, formamide, N-methylformamide, lauramide and N-methylbenzamide.

As characterized above, this process is operated as a homogeneous liquid phase mixture. The reaction is preferably operated in an inert solvent. Preferred inert solvents are those which permit at least partial

dissolution of the cobalt catalyst precursor, the amide and the aldehyde compound. These are generally polar solvents, for example of the ester, ether, ketone, amide, sulfoxide or aromatic hydrocarbon type.

Methyl and ethyl acetate are examples of suitable solvents. Other polar solvents are ethers, such as p-dioxane, methyl tertiary butyl ether, methyl tertiary amyl ether or tetrahydrofuran, tertiary amides, such as dimethyl formamide, dimethyl sulfoxide and ethylene carbonate.

In Examples V - VII no succinonitrile is used, and the solvents are varied using N,N-dimethylacetamide, methanol and acetonitrile. The yield of N-acetylglycine was either none or very insignificant.

The preferred solvent is ethyl acetate.

The N-acetylglycine is often insoluble in the solvent phase. This permits separation of the cobalt catalyst which may dissolve into the solvent phase, with or without prior acidification.

The quantity of cobalt-containing compound, succinonitrile promoter, and solvent to be used in the first embodiment of the invention may vary. The process is conducted in the presence of a catalytically effective quantity of the active cobalt-containing compound, succinonitrile promoter and solvent which gives the desired product in reasonable yield. The reaction proceeds when employing as little as 0.1 weight percent, and even lesser amounts of the cobalt-containing compound, along with as little as 0.1 weight percent of the succinonitrile promoter based on the total weight of the reaction mixture. The upper concentration is dictated by a variety of factors including catalyst cost, partial pressures of carbon monoxide and hydrogen and operating temperature. A cobalt-containing compound concentration of from 0.1 to about 10 weight percent in conjunction with a succinonitrile concentration of from 0.1 to 10 percent and an inert solvent concentration of from 10 to about 80 percent, based on the total weight of the reaction mixture is generally desirable in the practice of this invention.

Particularly superior results are obtained when the above-noted components of the catalyst system are combined as follows on a molar basis: Cobalt-containing compound to succinonitrile of 1.0 : 0.1 to 1.0 : 5.0.

The quantity of cobalt-containing compound, tertiary phosphine and solvent to be used in the second embodiment of the invention may also vary. The process is conducted in the presence of a catalytically effective quantity of the active cobalt-containing compound, tertiary phosphine and solvent which gives the desired product in reasonable yield. The reaction proceeds when employing as little as 0.1 weight percent, and even lesser amounts of the cobalt-containing compound, along with as little as 0.1 weight percent of the tertiary phosphine, such as N-tributylphosphine, based on the total weight of the reaction mixture. The upper concentration is dictated by a variety of factors including catalyst cost, partial pressures of carbon monoxide and hydrogen, operating temperature, etc. A cobalt-containing compound concentration of from 0.1 to 10 weight percent in conjunction with a N-tributylphosphine concentration of from 0.1 to 10 percent and an inert solvent concentration of from 10 to 80 percent, based on the total weight of the reaction mixture is generally desirable in the practice of this invention.

Particularly superior results are obtained when the above-noted components of the catalyst system are combined as follows on a molar basis: Cobalt-containing compound to trialkylphosphine of 1.0 : 0.1 to 1.0 : 5.0.

The operating conditions may vary over a wide range. The reaction temperature may vary from 25°C to 300°C. The preferred temperature is from 80°C to 150°C. The pressure may range from 3.5 MPa (500 psi) to 27.56 MPa (4000 psi) or more, however, the preferred pressure is from about 7 MPa (1000 psi) to about 27.56 MPa (4000 psi) in the embodiment using nitrile and less than 7 MPa (1000 psi) using the tributylphosphine ligand.

The amidocarbonylation reaction of this invention is best conducted in a carbon monoxide-rich atmosphere, although some hydrogen gas should also be present in order to achieve maximum cobalt catalyst activity. The hydrogen to carbon monoxide molar ratio in the reactor may be varied, for example, within the range from 20 : 1 to 1 : 20, but preferably it should be rich in carbon monoxide and the $H_2$ : CO ratio should be in the range 1 : 1 to 1 : 5.

The carbon monoxide employed need not satisfy particular purity requirements although catalyst contaminants should be avoided if the reaction is intended to continue over an extended period. Particularly in continuous operations, but also in batch experiments, the carbon monoxide and hydrogen gas may also be used in conjunction with up to 10 % by volume of one or more other gases. These other gases may include one or more inert gases such as argon and nitrogen or they may include gases that may, or may not, undergo reaction under carbon monoxide hydrogenation conditions, such as carbon dioxide, hydrocarbons, such as methane, ethane and propane, ethers, such as dimethyl ether, methyl ethyl ether and diethyl ether, alkanols, such as methanol.

In all these synthesis in order to achieve a high degree of selectivity the amount of carbon monoxide, aldehyde and amide present in the reaction mixture should be sufficient to at least satisfy the stoichiometry of-the desired formation of N-acetylglycine as shown in Equation 1 above. Excess carbon monoxide over the stoichiometric amount may be present and is desirable.

The desired products of this synthesis are N-acylaminoacids. The main desired product, N-acetylglycine, will be formed in significant quantities, with yields of up to 80 % in the embodiment using succinonitrile and as high as 75 % in the presence of a tertiary phosphine such as N-tributylphosphine. Also formed are significant amounts of bisamidal, a condensation product of paraformaldehyde and acetamide. Each of these products, including byproducts can be recovered from the reaction mixture by, conventional means, e. g. recrystallization.

The novel process of the invention can be conducted in a batch, semi-continuous or continuous manner. The

catalyst can be initially introduced into the reaction zone batchwise, or it may be continuously or intermittently introduced into such a zone during the course of the synthesis reaction. Operating conditions can be adjusted to optimize the formation of the desired amino acid product, and said material may be recovered by methods known to the art, such as filtration, recrystallization distillation, extraction and the like. A fraction rich in the catalyst components may then be recycled to the reaction zone, if desired, and additional products generated.

The products have been identified in this work by one or more of the following analytical procedures: viz, gas-liquid phase chromatography (glc), gas chromatography/infrared spectroscopy (GC/IR), nuclear magnetic resonance (nmr) and elemental analysis, or a combination of these techniques. Analysis have for the most part, been by molar weight; all temperatures are in degrees centigrade and all pressures in MPal additionally in pounds per square inch (psi).

The yield of N-acetylglycine in each synthesis (mole %) is estimated basis equation 1 using the formula:

$$\frac{\text{Moles of N-Acetylglycine Obtained}}{\text{Moles of Paraformaldehyde Charged}} \times 100\ \%$$

To illustrate the process of the invention, the following examples I, V, VI, and XII - XV and comparative exemples II - IV, VII - IX and XI are given.

## Exemple I

A glass-lined autoclave was charged with dicobalt octacarbonyl (0.34g, 1.0 mole), succinonitrile (0.08g, 1.0 mmole), paraformaldehyde (2.0 g, 0.066 mole), acetamide (5.99, 0.1 mole) and ethyl acetate (15.0 g). The reactor was sealed and flushed with the mixture of $CO/H_2$ 1 : 1 molar ratio, then pressured with $CO/H_2$ (1 : 1) to 8.27 MPa (1200 psi) and CO to 15.85 MPa (2300 psi) (resulting ca. 3 : 1 $CO/H_2$ mixture). The system was heated to 120°C and the maximum pressure of 20.67 MPa (3000 psi) was recorded during the process. The reaction was run for 2 hours; then the reactor was cooled to room temperature and the excess gas was vented. The product material was filtered. Solid material (8.5 g) and liquid (15.7g) were recovered. The H-nmr showed two products: N-acetylglycine (1) (81 % yield basis on paraformaldehyde charged) and bisamidal, (2), (6 % yield).

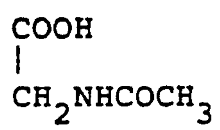

(1)

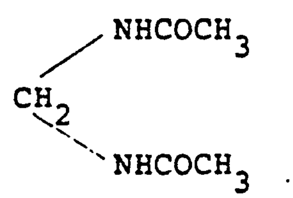

(2)

The cobalt analysis of the liquid showed 3470 ppm concentration, 47 % cobalt recovered basis dicobalt octacarbonyl charged.

## Exemples II - IX

In Examples II - IX the same procedure was used as was used in Example I.

Table I below shows data for Examples II - IX.

**Table I**

| Example | Ligand | Solvent | Reaction Conditions | Product Ratio (I) (II) (by mole %) | Yield of (I) (%) |
|---|---|---|---|---|---|
| II | None | EtOAc 15 g | $CO/H_2$ = 3 : 1 18.43 MPa 120°C 2 hr | 77 : 23 | 63 |
| III | None | EtOAc 15 g | $CO/H_2$ = 3 : 1 19.98 MPa 120°C 2 hr | 80 : 20 | 58 |
| IV | TMEDA 0.116 g 1 mm | EtOAc 15 g | $CO/H_2$ = 3 : 1 19.98 MPa 120°C 2 hr | 0 : 100 | 0 |
| V | Succino-nitrile 3.0 mmoles | EtOAc 15g | $CO/H_2$ = 3 : 1 19.98 MPa 120°C 2 hr | 100 : 0 | 78 |
| VI | Succino-nitrile 6.0 mmoles | EtOAc 15 g | $CO/H_2$ = 3 : 1 19.98 MPa 120° 2 hr | 94 : 6 | 76 |
| VII | None | N,N-dimethyl acetamide 15 g | $CO/H_2$ = 3 : 1 19.98 MPa 120°C 2 hr | 0 : N.D. | 0 |
| VIII | None | Methanol 15 g | $CO/H_2$ = 3 : 1 19.98 MPa 120°C 2 hr | 0 : N.D. | 0 |
| IX | None | Aceto-nitrile 15 g | $CO/H_2$ = 3 : 1 19.98 MPa 120°C 2 hr | 66 : 34 | 27 |

Notes: Example I: Cobalt in solution 3470 ppm (47 %)
Example II: <5 ppm
Example III, IV and VI: N.D.
Example IV: 5670 ppm (76 %)
Example VII: 4210 ppm (64 %)
Example VIII: 5250 ppm (82 %)
Example IX: 2750 ppm (42 %)

$Co_2(CO)_8$ 0.340 g, paraformaldehyde (20 g), acetamide (5.9 g)

Examples II and III are comparative in that only a dicobalt octacarbonyl catalyst was used and no succinonitrile. These two examples demonstrates:

(1) Lower selective to N-acetylglycine

(2) Very low cobalt recovery in liquid phase.

In Exemple IV a TMEDA liquid (i. e. tetramethylethylenediamine) was used in combination with the dicobalt octacarbonyl and it was found that the TMEDA deactivated the cobalt catalyst and gave no carbonylation products.

In Examples V and VI the amount of succinonitrile was varied. The difference in yield appeared to be small; however, referring back to Example I, in conjunction with Examples V and VI, it would appear that a smaller concentration is more effective.

In Examples VII - IX three defferent solvent were used, i. e. N,N-dimethylacetamide, methanol and acetonitrile. The low yields of these reactions might be due to the large amount of complexing agent (solvents) which deactivated the cobalt catalyst under these reaction conditions.

**Example X**

A 300 ml stirred autoclave was charged with dicobalt octacarbonyl (0.68 g, 2 mmoles), tri-n-butylphosphine (0.40 g, 2 mmoles), paraformaldehyde (2.0 g, 0.66 mole), acetamide (5.9 g 1.0 mole) and ethyl acetate (20 g). The reactor was sealed and purged with $CO/H_2$ (molar ratio 1 : 1). Then the reactor was pressured to 0.7 MPa (100 psi) with $CO/H_2$ (1 : 1) and heated to 120°C. At this temperature, the system was pressured with pure carbon monoxide to 5.5 MPa (800 psi). During the reaction process, the pressure-uptake was observed and the pressure was maintained at 5.5 MPa (800 psi) by pressuring additional carbon monoxide to the system. The addition of carbon monoxide was repeated during 3 hour reaction period. Then the system was cooled to room

temperature. The product mixtures, approximately 6.0 g brown solid and 21.5 g liquid were obtained. The solid was analyzed by H-nmr and showed the product (I), N-acetylglycine was

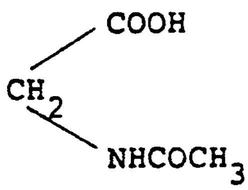 (I) 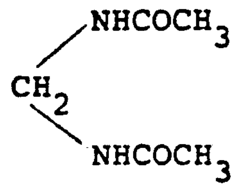 (II) EtOAc (III)

present at the molar ratio of 1.2 : 1.0 : 1.0 for compound I : II : III. The acetamide was present as a solid in relatively small quantity.

**Example XI** Comparative

Similar reaction procedures were used to those in Example I except for using mixtures of $CO_2(CO)_8$ (0.68 g), paraformaldehyde (2.0 g), acetamide (5.9 g) and ethyl acetate (20 g) and no tributylphosphine. During the similar reaction conditions, 120°C and 5.51 MPa (800 psi) carbon monoxide, there was no consumption of carbon monoxide observed. After three hours reaction time, a brown liquid product solution was obtained. The GC analysis showed no trace amount of N-acetylglycine produced.

The example demonstrated that n-$Bu_3P$ is important to activate cobalt catalyst at low CO pressure.

**Exemple XII**

The experimental procedures of Example I were used. Reaction mixtures of $Co_2(CO)_8$ (1.36 g), n-$Bu_3P$ (0.80 g), paraformaldehyde (4.0 g), acetamide (11.8 g) and methyl acetate (30 g) were used. After 2 hours reaction time, 39.5 g liquid plus 2.0 g solid materials were obtained. The solid products were analyzed by H-nmr, and showed compound 1 : 11 at a 10 : 1 molar ratio.

**Exemple XIII**

The experimental procedures of Example I were used. The reactor was charged with $Co_2(CO)_8$ (0.68 g), n-$Bu_3P$, (0.20 g), paraformaldehyde (2.0 g), acetamide (5.9 g) and ethyl acetate (20 g). The reaction conditions were 110 - 148°C, 2 hours, with the $CO/H_2$ ratio at approximately 2 : 1. The solid product mixture (approximately 4.0 g) was recovered. The H-nmr showed the ratio to be 1.0 : 1.8 : 1.9 of product I : II : III.

**Exemple XIV**

The experimental procedures of Example I were used. The reactor was charged with $Co_2(CO)_8$ (1.36 g), n-$Bu_3P$ (0.40 g), paraformaldehyde (2.0 g), acetamide (5.9 g) and ethyl acetate (20 g) The reaction conditions were 117°C, 4 hours, and a $CO/H_2$ ratio of approximately 8 : 1. The liquid (19.1 g) and solid (5.3 g) were obtained. Analysis showed the presence of compound I in the solid material.

**Exemple XV**

The experimental procedures of Example I were used. The reactor was charged with $Co_2(CO)_8$ (1.10 g), n-$Bu_3P$ (0.65 g), paraformaldehyde (2.0 g), acetamide (5.9 g), and ethyl acetate (20 g). The reaction conditions were $CO/H_2$ = 8 : 1 ratio, 101 - 109°C and 5 hours. The product mixtures contained 6.2 g solid material and approximately 21 g liquid. The H-nmr of solid material showed the molar ratio to be 19.0 : 1.0 : 3.5 of product I II : III. The yield of N-acetylglycine was estimated to be ca. 70 - 75 % based on paraformaldehyde charged.

**Claims**

1. A process for the production of N-acyl-L-amino acids of the formula

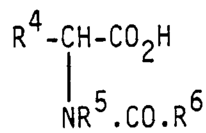

in which $R^4$, $R^5$ and $R^6$, which may be the same or different, are each hydrogen or a linear, branched or cyclic organic group, by reaction between an aldehyde of the formula

$R^4$-CHO

and an amide of the formula

$R^5$NH.CO.$R^6$

at a temperature of at least 25°C and a pressure of at least 3.5 MPa with carbon monoxide and hydrogen, in the presence of a cobalt catalyst, characterised in that the cobalt catalyst is promoted in an inert solvent by a tertiary phosphine of the formula

$R_3P$

where R represents an alkyl, aryl or alkaryl radical, or by a nitrile of the formula NC-$R^7$, -CN where $R^7$ represents an alkylene radical or an aromatic group.

2. A process as claimed in Claim 1, characterised in that the amide has the formula

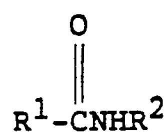

where the $R^1$ and $R^2$ groups are alkyl, aryl, arylalkyl and alkylaryl radicals or hydrogen.

3. A process as claimed in Claim 1 or 2, characterised in that the aldehyde is an aliphatic, alicyclic, aromatic or heterocyclic aldehyde.

4. A process according to any preceding Claim wherein $R^7$ an unbranched alkylene radical.

5. A process according to any preceding Claim characterised in that the aldehyde is paraformaldehyde.

6. A process according to any preceding Claim, characterised in that the amide is acetamide.

7. A process according to any preceding Claim, characterised in that the solvent is ethyl acetate or p-dioxane.

8. A process according to any preceding Claim, characterised in that the temperature is from 80 to 150°C.

9. A process according to any preceding Claim, characterised in that the catalyst is promoted by a dicarbonylic acid dinitrile and the pressure is from 7 to 28 MPa.

10. A process according to Claim 9, characterised in that the dicarbonylic acid dinitrile is succinonitrile.

11. A process according to any of Claims 1 to 8, characterised in that the catalyst is promoted by the tertiary phosphine and the pressure is from 3.5 to 7 MPa.

12. A process according to Claim 11, characterised in that the tertiary phosphine is tributyl phosphine.

**Patentansprüche**

1. Verfahren zur Herstellung von N-Acyl-L-aminosäuren der Formel

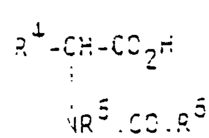

worin $R^4$, $R^5$ und $R^6$, die gleich oder verschieden sein können, jeweils für Wasserstoff oder eine lineare, verzweigte oder cyclische organische Gruppe stehen, durch Umsetzung zwischen einem Aldehyd der Formel

$R^4$-CHO

und einem Amid der Formel

$R^5NH.CO.R^6$

bei einer Temperatur von mindestens 25°C und einem Druck von mindestens 3,5 MPa mit Kohlenmonoxyd und Wasserstoff in Gegenwart eines Kobaltkatalysators, dadurch gekennzeichnet, daß der Kobaltkatalysator in einem inerten Lösungsmittel mit einem tertiären Phosphin der Formel

$R_3P$

worin R für einen Alkyl-, Aryl- oder Alkarylrest steht, oder mit einem Nitril der Formel $NC\text{-}R^7\text{-}CN$, worin $R^7$ für einen Alkylenrest oder eine aromatische Gruppe steht, aktiviert ist.

2. Verfahren nach Anspruch 1, dadurch gekennzeichnet, daß das Amid der Formel

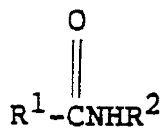

worin die $R^1$- und $R^2$-Gruppen Alkyl-, Aryl-, Arylalkyl- und Alkylarylreste oder Wasserstoff darstellen, entspricht.

3. Verfahren nach Anspruch 1 oder 2, dadurch gekennzeichnet, daß der Aldehyd ein aliphatischer, alicyclischer, aromatischer oder heterocyclischer Aldehyd ist.

4. Verfahren nach einem der vorhergehenden Ansprüche, worin $R^7$ einen unverzweigten Alkylenrest darstellt.

5. Verfahren nach einem der vorhergehenden Ansprüche, dadurch gekennzeichnet, daß der Aldehyd Paraformaldehyd ist.

6. Verfahren nach einem der vorhergehenden Ansprüche, dadurch gekennzeichnet, daß das Amid Acetamid ist.

7. Verfahren nach einem der vorhergehenden Ansprüche, dadurch gekennzeichnet, daß das Lösungsmittel Ethylacetat oder p-Dioxan ist.

8. Verfahren nach einem der vorhergehenden Ansprüche, dadurch gekennzeichnet, daß die Temperatur 80 bis 150°C beträgt.

9. Verfahren nach einem der vorhergehenden Ansprüche, dadurch gekennzeichnet, daß der Katalysator mit einem Dicarbonsäuredinitril aktiviert ist und der Druck 7 bis 28 MPa beträgt.

10. Verfahren nach Anspruch 9, dadurch gekennzeichnet, daß das Dicarbonsäuredinitril Bernsteinsäuredinitril ist.

11. Verfahren nach einem der Ansprüche 1 bis 8, dadurch gekennzeichnet, daß der Katalysator mit einem tertiären Phosphin aktiviert ist und der Druck 3,5 bis 7 MPa beträgt.

12. Verfahren nach Anspruch 11, dadurch gekennzeichnet, daß das tertiäre Phosphin Tributylphosphin ist.

**Revendications**

1. Procédé de production des N-acyl-L-aminoacides de la formule

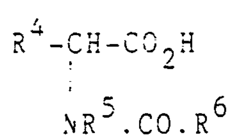

dans laquelle $R^4$, $R^5$ et $R^6$ qui peuvent être les mêmes ou différents, sont, chacun, de l'hydrogène ou un groupe organique à chaîne linéaire, ramifiée ou cyclique, par réaction entre un aldéhyde de formule

$R^4\text{-}CHO$

et un amide de formule

$R^5NH.CO.R^6$

à une température d'au moins 25°C à une pression d'au moins 3,5 MPa avec de l'oxyde de carbone et de l'hydrogène, en présence d'un catalyseur à base de cobalt, caractérisé en ce que le catalyseur à base de cobalt est active dans un solvant inerte par une phosphine tertiaire de formule

$R_3P$

où R représente un radical alcoyle, aryle ou alcaryle, ou par un nitrile de formule NC-$R^7$-CN ou $R^7$ représente un radical alcoylène ou un groupe aromatique.

2. Procédé selon la revendication 1, caractérisé en ce que l'amide a la formule

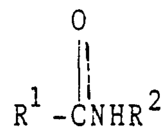

où les groupes $R^1$ et $R^2$ sont des radicaux alcoyle, aryle, arylalcoyle et alcoylaryle ou de l'hydrogène.

3. Procédé selon la revendication 1 ou 2, caractérisé en ce que l'aldéhyde est un aldéhyde aliphatique alicyclique aromatique ou hétérocyclique.

4. Procédé selon l'une quelconque des revendications précédentes dans lequel $R^7$ est un radical alcoylène non ramifié.

5. Procédé selon l'une quelconque des revendications précédentes caractérisé en ce que l'aldéhyde est un para formaldéhyde.

6. Procédé selon l'une quelconque des revendications précédentes caractérisé en ce que l'amide est un acétamide.

7. Procédé selon l'une quelconque des revendications précédentes caractérisé en ce que le solvant est de l'acétate d'éthyle ou du p-dioxane.

8. Procédé selon l'une quelconque des revendications précédentes, caractérisé en ce que la température va de 80 à 150°C.

9. Procédé selon l'une quelconque des revendications précédentes, caractérisé en ce que le catalyseur est active par un dinitrile acide dicarbonylé et en ce que la pression va de 7 à 28 MPa.

10. Procédé selon la revendication 9, caractérisé en ce que le dinitrile acide dicarbonylé est le succinonitrile.

11. Procédé selon l'une quelconque des revendications 1 à 8, caractérisé en ce que le catalyseur est activé par la phosphine tertiaire et en ce que la pression va de 3,5 à 7 MPa.

12. Procédé selon la revendication 11, caractérisé en ce que la phosphine tertiaire est la tributylphosphine.